## Europäisches Patentamt
## European Patent Office
## Office européen des brevets

(11) Veröffentlichungsnummer: **0 141 051**
**A2**

# EUROPÄISCHE PATENTANMELDUNG

(21) Anmeldenummer: **84108640.8**

(22) Anmeldetag: **21.07.84**

(51) Int. Cl.⁴: **A 61 K 9/48**
**A 61 K 47/00**

(30) Priorität: 16.08.83 DE 3329440
13.10.83 DE 3337186
02.05.84 DE 3416162
24.04.84 DE 3415250

(43) Veröffentlichungstag der Anmeldung:
15.05.85 Patentblatt 85/20

(84) Benannte Vertragsstaaten:
AT BE CH DE FR GB IT LI LU NL SE

(71) Anmelder: Ismail, Roshdy, Dr.
Siebengebirgs-Apotheke Siebengebirgsallee 2
D-5000 Köln 41 (Klettenberg)(DE)

(72) Erfinder: Ismail, Roshdy, Dr.
Siebengebirgs-Apotheke Siebengebirgsallee 2
D-5000 Köln 41 (Klettenberg)(DE)

(74) Vertreter: Werner, Hans-Karsten, Dr. et al,
Deichmannhaus am Hauptbahnhof
D-5000 Köln 1(DE)

(54) **Weichgelatinekapseln enthaltend Vitamin A und E.**

(57) Weichgelatinekapseln enthaltend Vitamin A und E, enthaltend zusätzlich in der Füllung neutrale Öle und Polysorbat 80. Sie sind stabil, gut einnehmbar, gut absorbierbar und besser verträglich als bisher bekannte Rezepturen.

EP 0 141 051 A2

Gegenstand der vorliegenden Erfindung sind Weichgelatinekapseln, enthaltend Vitamin A und E.

Es sind Weichgelatinekapseln bekannt, welche pro Kapsel
beispielsweise 100 mg Vitamin E (alpha-Tocopherol) aus
Soja-, Mais- oder Weizenkeimlingen und 60 mg Weizenkeimöl enthalten. Es wird empfohlen, von diesen Kapseln
täglich morgens eine unzerkaut vor der Mahlzeit einzunehmen. Bei größerer Belastung sollen je eine weitere
Kapsel mittags und abends eingenommen werden.

Weiterhin bekannt ist eine Emulsion, welche pro ml 16,5
mg Retinolpalmitat (entsprechend 30.000 I.E. Vitamin A)
und 75, mg D,L-alpha-Tocopherolacetat (Vitamin-E-acetat) enthalten. Von dieser Emulsion sollen 2 mal täglich 10 bis 15 Tropfen zum Frühstück und Abendessen
eingenommen werden, woraus sich eine Tagesdosis bis zu
45.000 I.E. Vitamin A ergibt.

Genauere Untersuchungen bezüglich der Resorption und
Wirksamkeit von Vitamin E haben ergeben, daß ein Großteil des Vitamin E durch die Magensäure zerstört wird,
so daß selbst bei einer täglichen Dosierung von 300 bis
400 mg höchstens 200 mg in wirksamer Form aufgenommen
werden können; vgl. Arthur Vogelsang in Angiology 21,
275-79 (1970). In dieser Untersuchung wird beschrieben,
daß die Dosierung bei Coronarinsuffizienz nicht unter
300 ml pro Tag absinken sollte.

Die vorliegende Erfindung hat sich die Aufgabe gestellt, ein Vitamin-E-Präparat mit einem Gehalt an Vitamin A zu entwickeln, welches leicht einnehmbar,
leicht absorbierbar, gut verträglich und lagerfähig
ist, eine konstante Dosierung ermöglicht und dabei eine
ausreichend hohe Dosierung insbesondere an Vitamin E
gewährleistet, ohne dabei zu hohe Dosierungen an Vita-

- 3 -  0141051

min A zu bewirken. Kapseln mit einem hohen Gehalt an Vitamin E und Vitamin A sind bisher nicht bekannt.

Diese Aufgabe wurde überraschenderweise gelöst durch Weichgelatinekapseln enthaltend

60 bis 93 Gew.-% Vitamin E,
1,8 bis 11 Gew.-% Vitamin A,
4 bis 30 Gew.-% neutrale Öle und
0,1 bis 5 Gew.-% Emulgator.

Das Vitamin E kann in seinen alpha-Formen als freies Tocopherol oder Ester vorliegen. Das Vitamin E wird dabei vorzugsweise als D,L-alpha-Tocopherolacetat oder natürliches D-alpha-Tocopherolacetat eingesetzt. Als Vitamin A wird vorzugsweise Vitamin A-Palmitat eingesetzt. Als Neutralöle kommen Sojaöl oder Erdnußöl oder Gemische in derselben in Frage. Weiterhin müssen die Kapseln einen Emulgator enthalten, und zwar in einer Menge von 0,1 bis 5 Gew.-%, vorzugsweise 0,4 bis 5 Gew.-%. Ganz besonders bevorzugt wird 1 Gew.-%.

Die Füllung von bisher bekannten Weichgelatinekapseln enthaltend Vitamin E in Öl, enthielten maximal 62,5 Gew.-% Vitamin E. Erfindungsgemäß ist es möglich, den Gehalt an Vitamin E der Füllung auf 60 bis 93 Gew.-%, vorzugsweise 70 bis 75 Gew.-%, ggf. aber auch 70 bis 85 Gew.-% zu erhöhen und dabei zusätzlich eine bessere Verträglichkeit und leichtere Resorption zu erzielen. Dies scheint darauf zurückzuführen sein, daß die Rezeptur zusätzlich 1,8 bis 11, vorzugsweise 4 bis 5,5 Gew.-% Vitamin A enthält sowie 0,4 bis 5 Gew.-%, vorzugsweise 1 bis 5 Gew.-% Emulgator. Ganz besonders wird die Resorption bei Gegenwart von 1 Gew.-% Emulgator erleichtert. Es wurde überraschenderweise gefunden, daß Emulgatoren in so geringen Konzentrationen, die Löslichkeit von Vitamin E und Vitamin A in Ölen erhöht und daß derartige Gemische die vollständige und rasche Absorption des Vitamin E und A bewirken. Hieraus resultiert auch eine bessere Verträglichkeit und erhöhte Wirksamkeit. Dieser Effekt war nicht vorhersehbar, da man bisher bestrebt war, diese Effekte durch einen möglichst großen Emulgatoranteil zu erzielen. Neuere eigene Untersuchungen haben ergeben, daß Emulgatoranteile von mehr als 5 Gew.-% nicht diese Effekte zur Folge haben oder aber zu trüben Gemischen führen, aus denen Einzelbestandteile sedimentieren, so daß sie sich nicht in Kapseln füllen lassen oder zu instabilen Kapseln führen. Besonders bevorzugte Emulgatoren sind Ethoxylate von Fettalkoholen, hydriertem Ricinusöl und Nonylphenol und Ester mittel- bis langkettiger Fettalkohole, Cetylstearylalkohol, aliphatische Partialester der Triglyceride oder mehrwertige Alkohole wie Sorbit oder Mannit. Ganz besonders bevorzugt wird Polysorbat 80. Eigene Untersuchungen haben gezeigt, daß mit Polysorbat 80 die genannten erfindungsgemäßen Effekte besonders gut sind.

Die erfindungsgemäßen Kombinationen verbessern außerdem die Durchblutung der Extremitäten, der Peripherie des Auges, des Innenohres und des Cerebrum und sind daher zur Behandlung der Arteriosklerose geeignet. Die Wirksamkeit bei diesen Indikationen ist besonders überraschend und ermöglicht weitere neue Anwendungsgebiete.

Diese Effekte waren auch nicht herleitbar aus der handelsüblichen Emulsion von Vitamin A und Vitamin E. Von dieser Zubereitungsform mußte eine wesentlich höhere Menge eingenommen werden, um den erforderlichen Bedarf an Vitamin E zu decken. Hierbei gerät man jedoch sehr rasch in den Bereich, in dem das Vitamin A überdosiert wird. Außerdem war diese Emulsion in der Praxis nicht genau dosierbar und unangenehm einzunehmen. Auch die Stabilität angebrochener Packungen ließ zu wünschen übrig. Außerdem verklumpen die Emulsionen aus Vitamin A und E leicht in Wasser, so daß die Verluste an Vitamin A sehr groß werden.

Die erfindungsgemäßen Weichgelatinekapseln lassen sich ohne Schwierigkeiten in Größen von 200 bis 900 mg Rohgewicht verarbeiten. Da eine Dosierung von 200 bis 500 mg, vorzugsweise 200 bis 400 mg Vitamin E angestrebt wird, bieten sich Rohgewichte von 280 bis 700 mg Weichgelatinekapseln an. Da die Gelatinehülle derartiger Kapseln etwa 100 bis 200 mg wiegt, erhält man so Kapseln mit einem Gewicht von ca. 400 bis 900 mg, die erfahrungsgemäß sehr gut eingenommen werden können.

Als Neutralöl kann Soja- und/oder Maisöl in einer Menge von mindestens 4 bis 5 Gew.-% verwendet werden. Im allgemeinen beträgt der Anteil der Neutralöle 4 bis 30 Gew.-%, vorzugsweise 4 bis 22 Gew.-%. Besonders bevorzugt werden 18 bis 20 Gew.-%. Es können auch Gemische aus anderen Stoffen, z.B. Vitamine, wie Vitamin $B_1$, $B_2$ und $B_6$ oder Nicotinsäure bzw. deren Ester oder Derivate oder B-Hydroxyrutosid sowie andere geeignete Stoffe zugesetzt werden.

Die Weichgelatinehülle kann zusätzlich noch übliche Farbstoffe und Zusätze enthalten. Es kommen die Standardrezepturen für Weichgelatinekapseln in Frage wie sie beispielsweise von der Firma R.P. Scherer GmbH, Eberbach entwickelt und angewendet werden.

Eine bevorzugte Ausführungsform der erfindungsgemäßen Weichgelatinekapseln ist in dem nachfolgenden Beispiel

- 6 -     **0141051**

beschrieben.

### B e i s p i e l  1

Die Füllung der Weichgelatinekapsel enthält Vitamin A-Palmitat (Ph.Eur.III, USP 13,75 bis 15,125 mg = 25.000 bis 27.500 I.E. Vit. A)
200 bis 210 mg DL-alpha-Tocopherolacetat (Ph.Eur.III USP)
Sojaöl USP 42,5 mg
Erdnußöl DAB, NF 12,375 mg
Polysorbat 80 (Ph.Eur.III = Tween 80) 10 mg.

Diese Füllung wurde mit einem Gelatineöl von 115,58 mg ± 8% = 106,33 bis 124,83 mg überzogen. Die Weichgelatinehülle enthielt 75 bis 88 mg Gelatine USP, DAB, 21 mg Glyzerin 85%-ig und 12 bis 14 mg Trockenmasse bestehend aus Sorbit, Sorbitan und Mannit.

### B e i s p i e l  2

Die Füllung der Weichgelatinekapsel enthält Vitamin A-Palmitat (Ph.Eur.III, USP 27,5 mg = 50.000 I.E. Vit. A) 400 bis 410 mg DL-alpha-Tocopherolacetat (Ph.Eur.III USP), 70 mg Sojabohnenöl, 24,77 mg Erdnußöl und 15 mg Polysorbat 80 (Ph.Eur.III = Tween 80). Das in diesem Beispiel verwendete Vitamin E ist natürlicher Herkunft (1100 I.E./g).

Diese Füllung wurde mit dem Gelatineöl gemäß Beispiel 1 überzogen.

### B e i s p i e l  3

Die Füllung der Weichgelatinekapsel enthält Vitamin A-Palmitat (Ph.Eur.III, USP 50.000 I.E. bis 25.500 I.E., entspricht ca. 27,5 mg reinem Vitamin A), 200 bis 210 mg DL-alpha-Tocopherolacetat (Ph.Eur.III USP), 60 mg Sojabohnenöl, 27,5 mg Erdnußöl und 12 mg Polysorbat 80 (Tween 80).

Diese Füllung wurde mit dem Gelatineöl gemäß Beispiel 1 überzogen.

B e i s p i e l 4

Die Füllung der Weichgelatinekapsel enthält Vitamin A-Palmitat (Ph.Eur.III, USP 13,75 bis 15,125 mg = 25.000 bis 27.500 I.E. Vitamin A), 400 bis 410 mg DL-alpha-Tocopherolacetat (Ph.Eur.III USP), 100 mg Sojabohnenöl, 12,37 mg Erdnußöl und 21 mg Polysorbat 80 (Tween 80).

Diese Füllung wurde mit dem Gelatineöl gemäß Beispiel 1 überzogen.

B e i s p i e l 5

Die Füllung der Weichgelatinekapsel enthält Vitamin A-Palmitat (Ph.Eur.III, USP 13,75 bis 15,25 mg = 25.000 bis 27.500 I.E. Vitamin A), 400 bis 410 mg DL-alpha-Tocopherolacetat (Ph.Eur.III USP), 40 mg Sojabohnenöl, 12,73 mg Erdnußöl, 10 mg Polysorbat 80 (Tween 80). Diese Füllung wurde mit dem Gelatineöl gemäß Beispiel 1 überzogen.

B e i s p i e l 6

Die Füllung der Weichgelantinekapsel enthält

400 mg D-alpha-Tocopherol-Konzentrat,
42,5 mg Sojaöl,

13,75 mg bis 15,125 mg = 25.000 bis 27.500 I.E., Vitamin A Palmitat, 12,375 mg Erdnußöl, 4 mg Polysorbat 80 (Tween 80).

Diese Füllung wurde mit dem Gelatineöl gemäß Beispiel 1 überzogen.

B e i s p i e l 7

Gemäß Beispiel 6 wurden 2 mg Polysorbat 80 verwendet.

B e i s p i e l 8

Diese Füllung der Weichgelatinekapsle enthält

400 bis 410 mg D-alpha-Tocopherolacetat, 52 mg Sojaöl, 27,5 mg Vitamin A Palmitat, 25 mg Erdnußöl,

4 mg Ölsäureoleylester (Cetiol$^R$).

Diese Füllung wurde mit dem Gelatineöl gemäß Beispiel 1 überzogen.

B e i s p i e l 9

Gemäß Beispiel 8 wurden 1,5 Cetiol$^R$ anstelle von 4 mg verwendet.

B e i s p i e l 10

Gemäß Beispiel 8 wurden

4 mg Polysorbat 80 anstelle von Cetiol oder
4 mg Cremophor$^R$ anstelle von Cetiol verwendet.

Unter dem Warenzeichen Cremophor wird ein Sortiment von flüssigen bis pastösen oder wachsartigen Emulgatoren und Lösungsvermittlern für Kosmetik und Pharmazie vertrieben. Es handelt sich hierbei um Ethoxylate von Fettalkoholen bzw. hydriertem Ricinusöl bzw. Nonylphenol, die als nichtionogene Emulgatoren zur Herstellung von Öl-in-Wasser-Emulsionen für Cremes bzw. zur Solubilisierung von Parfümölen und Vitaminen in kosmetischen Produkten dienen.

B e i s p i e l   11

Die Füllung der Weichgelatinekapsel enthält

500 bis 510 mg DL-alpha-Tocopherolacetat, 27,5 mg = 50.000 I.E. Vitamin A Acetat, 90 mg Sojaöl, 5 mg Polysorbat 80 (Tween 80), 24,77 mg Erdnußöl, je 5 mg von Vitamin $B_1$, $B_2$, $B_6$,

5 µg Vitamin B 12, 15 mg Nicotinsäureamid.

Diese Füllung wurde mit Gelatineöl gemäß Beispiel 1 überzogen.

B e i s p i e l   12

Gemäß Beispiel 11 wurde der gleiche Ansatz, jedoch mit Vitamin A Palmitat anstelle von Acetat durchgeführt.

B e i s p i e l   13

Gemäß Beispiel 8 wurde 1 mg Polysorbat 80 anstelle von 4 mg Cetiol$^R$ verwendet.

## B e i s p i e l  14

Die Füllung der Weichgelatinekapsel enthält

200 bis 210 mg DL-alpha-Tocopherolacetat, 42,5 mg Sojaöl, 12,375 mg Erdnußöl, 2 mg Polysorbat 80.

Diese Füllung wurde mit Gelatineöl gemäß Beispiel 1 überzogen.

## B e i s p i e l  15

Die Füllung der Weichgelatinekapsel enthält

200 bis 219 mg DL-alpha-Tocopherolacetat, 70 mg Sojaöl, 12,4 mg Erdnußöl, 2 mg Polysorbat 80, je 5 mg Vitamin $B_1$, $B_2$, $B_6$,

5 µg B 12, 15 mg Nicotinsäureamid.

Diese Füllung wurde mit Gelatineöl gemäß Beispiel 1 überzogen.

Die so erhaltenen Kapseln sind lagerstabil, gut einnehmbar, leicht absorbierbar, gut verträglich und entwickeln eine höhere Wirksamkeit als bisher bekannte Zubereitungen von Vitamin E.

## B e i s p i e l  16

Vitamin A Palmitat 15.000 I.E.,

DL-alpha-Tocopherolacetat 400 mg, Magnesiumorotat        200 mg,

| Magnesiumaspartat | 200 mg, |
| Polysorbat 80 | 4 mg. |

## Beispiel 17

Vitamin A Palmitat 15.000 I.E.,
DL-alpha-Tocopherolacetat 400 mg,
Magnesiumhydrogenphosphat 200 mg,

| Magnesiumaspartat | 150 mg, |
| Magnesiumcitrat | 50 mg |
| Polysorbat 80 | 4 mg. |

<u>Patentansprüche</u>

1.) Weichgelatinekapseln enthaltend

    60    bis 93    Gew.-% Vitamin E,
    1,8   bis 11    Gew.-% Vitamin A,
     4    bis 30    Gew.-% neutrale Öle und
   0,1 bis  5    Gew.-% Emulgator.

2.) Weichgelatinekapseln nach Anspruch 1, dadurch gekennzeichnet, daß die Füllung 70 bis 75 Gew.-% Vitamin E enthält.

3.) Weichgelatinekapseln nach einem der Ansprüche 1 oder 2, dadurch gekennzeichnet, daß die Füllung 4 bis 5,5 Gew.-% Vitamin A enthält.

4.) Weichgelatinekapseln nach einem der Ansprüche 1 bis 3, dadurch gekennzeichnet, daß die Füllung 18 bis 20 Gew.-% neutrale Öle enthält.

5.) Weichgelatinekapseln nach einem der Ansprüche 1 bis 4, dadurch gekennzeichnet, daß die Füllung 0,4 bis 5 Gew.-% Emulgator enthält.

6.) Weichgelatinekapseln nach einem der Ansprüche 1 bis 5, dadurch gekennzeichnet, daß die Füllung 1 Gew.-% Emulgator enthält.

7.) Weichgelatinekapseln nach einem der Ansprüche 1 bis 6, dadurch gekennzeichnet, daß als Emulgator ein Ethoxylat von Fettalkoholen, hydriertem Ricinusöl oder Nonylphenol, Ester mittel- bis langkettiger Fettalkohole sowie Polysorbat 80 verwendet werden.

- 13 -

0141051

8.) Weichgelatinekapseln nach einem der Ansprüche 1 bis 7, dadurch gekennzeichnet, daß das Vitamin E als DL-alpha-Tocopherolacetat oder als natürliches D-aplpha-Tocopherolacetat vorliegt.

9.) Weichgelatinekapseln nach einem der Ansprüche 1 bis 7, dadurch gekennzeichnet, daß das Vitamin A als Vitamin A-Palmitat vorliegt.

10.) Weichgelatinekapseln nach einem der Ansprüche 1 bis 9, dadurch gekennzeichnet, daß als neutrale Öle Sojaöl oder Erdnußöl oder Gemische derselben verwendet werden.